(19) Europäisches Patentamt
European Patent Office
Office européen des brevets

(11) **EP 4 052 643 B1**

(12) **EUROPEAN PATENT SPECIFICATION**

(45) Date of publication and mention of the grant of the patent:
**04.10.2023 Bulletin 2023/40**

(21) Application number: **21382183.8**

(22) Date of filing: **04.03.2021**

(51) International Patent Classification (IPC):
**A61B 5/0215** (2006.01)     **A61B 5/07** (2006.01)
**A61F 2/90** (2013.01)

(52) Cooperative Patent Classification (CPC):
**A61B 5/0028; A61B 5/0031; A61B 5/0215;**
**A61B 5/076; A61B 5/686; A61B 5/6876;**
**A61F 2/90;** A61B 5/02158; A61B 2560/0219;
A61B 2562/0247; A61B 2562/168; A61F 2240/001;
A61F 2250/0002

(54) **IMPLANTABLE SENSOR FOR MEASURING AND MONITORING INTRAVASCULAR PRESSURE, SYSTEM COMPRISING SAID SENSOR AND METHOD FOR OPERATING THEREOF**

IMPLANTIERBARER SENSOR ZUR MESSUNG UND ÜBERWACHUNG DES INTRAVASKULÄREN DRUCKS, SYSTEM MIT BESAGTEM SENSOR UND VERFAHREN ZUM BETRIEB DAVON

CAPTEUR IMPLANTABLE PERMETTANT DE MESURER ET DE SURVEILLER LA PRESSION INTRAVASCULAIRE, SYSTÈME COMPRENANT LEDIT CAPTEUR ET SON PROCÉDÉ DE FONCTIONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.09.2022 Bulletin 2022/36**

(73) Proprietor: **Universitat Pompeu Fabra**
**08002 Barcelona (ES)**

(72) Inventors:
• **IVORRA CANO, Antoni**
**08002 Barcelona (ES)**
• **BECERRA FAJARDO, Laura**
**08002 Barcelona (ES)**
• **COMERMA MONTELLS, Albert**
**08002 Barcelona (ES)**
• **MINGUILLON CAMPOS, Jesús**
**08002 Barcelona (ES)**

(74) Representative: **Tribalyte Ideas**
**Gta. Quevedo 8, 6°2**
**28015 Madrid (ES)**

(56) References cited:
**US-A1- 2009 171 413     US-A1- 2016 029 956**

• **Becerra Fajardo Laura ET AL: "Microcontrolled injectable stimulators based on electronic rectification of high frequency current bursts", , 17 October 2016 (2016-10-17), XP55829128, Retrieved from the Internet: URL:https://www.tdx.cat/bitstream/handle/1 0803/402433/tlbf.pdf?sequence=1&isAllowed= y [retrieved on 2021-07-30]**
• **Pascual Mar: "Roadmap for identifying clinical needs in technology-driven innovations: The Senso-eAXON case", , 1 July 2020 (2020-07-01), XP55829279, Retrieved from the Internet: URL:https://repositori.upf.edu/bitstream/h andle/10230/46527/Pascual_2020.pdf?sequenc e=1&isAllowed=y [retrieved on 2021-07-30]**

**Description**

**FIELD OF THE INVENTION**

The invention belongs to the field of active implantable medical devices (AIMDs). More specifically, the invention relates to an implantable pressure sensor for monitoring intravascular pressure, which can be powered and excited by volume conduction (also known as galvanic coupling).

**BACKGROUND OF THE INVENTION**

Nowadays, the use of implantable sensing systems is known in the clinical practice, so as to measure relevant magnitudes in a living body (either animal or human) like the blood pressure. Compared to external sensing systems, implantable systems can advantageously detect the stimuli closer to where they originate, thereby providing more accurate measurements. These implantable sensing systems are normally used for diagnosis and for determining treatment dosage and timing.

Millions of patients could benefit from continuous monitoring of biomedical parameters that currently can only be measured at discrete times. A remarkable case is that of intravascular pressure in heart failure monitoring. Regular pulmonary artery pressure measurements, along with clinical signs and symptoms, allow to improve the management of patients with heart failure, reducing the rate of hospitalization.

Non-invasive external measurement systems are obviously preferable over implantable or semi-implantable systems. However, for most biomedical parameters, external systems require the patient to perform some specific actions and hence do not allow continuous monitoring. In addition, since non-invasive external measurement systems typically rely on indirect parameters or sense the magnitudes away from the relevant inner body regions, they usually exhibit reliability and accuracy issues. All this explains the need for semi-implantable or implantable sensing systems. Semi-implantable systems consisting of an external electronic module connected to an implanted sensor or catheter are an option in some cases (e.g., glucose monitoring). However, semi-implantable systems are not feasible in other cases due to risk of infection (e.g., for intravascular pressure monitoring) and in those cases fully implantable systems are required.

A known type of electronic sensing implants is based on passive electronics, which do not include a mechanism to power the implant. Most of the commercially available sensors of this type are based on combinations of inductors and capacitors (LC systems) that resonate at a specific frequency when an alternating magnetic field is applied. Such frequency is typically determined by a capacitor which acts as the sensor, as its capacitance depends on the magnitude of interest (i.e., pressure). The main disadvantage of these systems is that they require coils with a relatively large diameter, both at the implant and at the external unit, especially if the device is intended for deep implantation.

As an alternative to passive electronics, other sensors are based on active electronics. These implants incorporate a mechanism to generate electric energy to power an electronic circuit capable of reading and processing signals from a sensor and transmitting the result to an external unit for further processing or graphical representation. In some cases, the electric power is entirely generated internally (e.g., with electrochemical batteries) and in other cases the electric power is either generated by transforming a source of energy already present in the body (e.g., the so-called energy harvesters that can transform kinematic energy into electric energy), or by wireless power transfer (WPT) from an external reading unit (e.g., by ultrasound power transmission or by inductive coupling power transmission).

Some wireless systems for interrogating intravascular sensors have been proposed and demonstrated in the past. For instance, patent application WO 2017200769 A2 refers to a method to power and interrogate implanted stents using a "touch probe" antenna (e.g., external electrodes) through a near-field electric connection, which makes direct contact with skin. Here the touch probe is always described as a planar (rectangular) and rigid structure and the excitation is provided continuously._However, this application is silent about hermeticity and the construction of a pressure sensor.

In the state of the art, the system CardioMEMS HF (manufactured by Abbott) is also known, which is a microelectromechanical sensor (MEMS) for monitoring pulmonary artery pressure in order to anticipate heart failure. The implant consists of a LC resonant circuit whose capacitance changes depending on the pressure. The size of this sensor is 45 mm x 3.4 mm x 2 mm. Nevertheless, the interrogation system of CardioMEMS requires a bulky paddle and a desktop system. Furthermore, CardioMEMS technology is adequate only for discrete measurements (e.g., once a day), which is an important limitation.

Furthermore, patent US 7,353,711 B1 discloses an electronic capacitive pressure sensor formed in a hermetically sealed cavity by using part of a housing as a diaphragm, thereby providing enough room for fitting sensing electronics (i.e., an integrated circuit, IC). However, the manufacturing of this sensor is rather complex.

More recently, so-called smart intravascular stents have been proposed by integrating various sensors for detecting vascular restenosis or monitoring medical conditions such as blood pressure or blood flow velocity, as the ones presented in the research article "A Single-Connector Stent Antenna for Intravascular Monitoring Applications" (Liu et al., published in Sensors, vol. 19, no. 21, p. 4616). The information about acquired pressure signals obtained with these sensors is then transmitted to external monitoring systems via wireless communications. In said article, a stent acting as an electrical inductor or antenna for signal transfer from integrated sensors to an external monitoring system is also disclosed, wherein the antenna consists of multiple conductive rings containing crowns and struts. Although this stent provides an antenna with good electromagnetic radiation efficiency, the placement of stent antenna with said rings might lead to complications during the surgical implantation. In this way, it would be preferable providing a stent which could be implanted without surgery. Moreover, embedding wide and rigid coils in the implants hampers the miniaturization. Patent application US 2016/0029956 A1 discloses wireless blood-vessel implants containing a housing, a pressure sensor, internal electronics, and thin wall comprising flexible membranes which communicate pressure to the internal electronics, by means of an incompressible fluid or gel that fills the cavity formed by the housing and the thin wall. The thin wall may be a flexible membrane which is part of a sensing electronic circuit, thus transducing pressure directly into an electronic signal of a sensing circuit. However, this document does not disclose any means for powering such implants but refers, instead, to passive sensors without any internal power supply elements. As such, for their operation they make use of elements such as LC resonant tank circuits, antennae, and coils.

The use of the volume conduction property of human tissues it is also known as a natural medium for the delivery of energy. For example, patent application WO 2006105245 A2 discloses the use of the volume conduction for energy delivery to implants, as an alternative to batteries. The disclosure of this application is focused on an external antenna design, which consists of an array of electrodes arranged to receive voltage and work collaboratively to transmit electrical energy to a target site. Under this arrangement, the external delivery of electrical energy from outside the human body to a target site within the human body is carried out providing electrical stimulation to muscles and delivering power to implanted devices. However, the application remains silent about the structure of the sensors and how said volume conduction currents must be processed. A further example of several systems comprising the use of volume conduction for energy delivery to skeletal muscle implants is disclosed in L. Becerra Fajardo et al., "Microcontrolled injectable stimulators based on electronic rectification of high frequency current bursts" (2016). However, this document does not propose any application or adaptation of these implants for their use in blood vessels or body cavities.

Furthermore, patent US 8,515,559 B2 refers exclusively to the use of volume conduction for communications in intravascular sensors. This document remains silent regarding the use of volume conduction for a purpose different from the communications. However, in the sensor disclosed in this patent, three major drawbacks are found:

1) The electrodes are always partially insulated, which can be counterproductive to obtain an effective power transfer.
2) There is an opening in the capsule of the sensor, which exposes a pressure transducer housed in the capsule. Said opening compromises the tightness of the capsule and makes it difficult to build the entire sensor to ensure that the hermeticity is maintained.
3) It refers to other powering methods which result bulky such as batteries.

Therefore, all the known implanted sensors (and more particularly, intravascular pressure sensors) present one or more of the following drawbacks:

- The implants have a short operational life because their power is based on batteries. Consequently, they are not adequate for long term implantation.
- The implants contain a mechanism for generating the electric energy requires bulky components that hinder miniaturization of the implants, in such a way that implantation by catheterization or injection is not feasible. That is to say, they are too large to be implanted in small vessels.
- The cross-section of the device to be implanted within the vessel's lumen is too large for smaller vessels (i.e., it would occupy an excessive portion of the lumen of the vessel).
- The electrical or electronic components of the implant are not protected within a hermetic package and hence are not adequate for long term implantation.
- It is not possible to obtain measurements from multiple nearby implants (i.e., the implants would mutually interfere). Therefore, each sensor is not selectively addressable, and so, one cannot operate simultaneously using multiple nearby sensors.
- The implants cannot be deployed at deep locations due to signal attenuation.

All the aforementioned problems are addressed, simultaneously, by the sensor, system and method proposed by this invention.

## BRIEF DESCRIPTION OF THE INVENTION

A first object of the invention is to provide an implantable intravascular pressure sensor to obtain reliable measurements of pressure related parameters, without requiring bulky powering systems (e.g., batteries) and therefore enabling miniaturization. This sensor provides measurements which may be clinically relevant per se (e.g., arterial blood pressure) or may be relevant for continuous monitoring the condition of a device, for instance, a therapeutic device attached to the pressure sensor (e.g., to monitor the pressure drop across a stent). This pressure sensor is adapted to be interrogated by a reading unit such that both devices cooperate to obtain reliable measurements of the pressure when the pressure sensor is deployed in a living body.

Preferably, this reading unit is configured for applying high-frequency bursts of alternating current to the tissues through the skin electrodes, and additionally, for interrogating the implant by modulating those bursts of high frequency electric currents. The measurement is obtained by processing the voltage or current signals that result in the electrodes during or after the delivery of the bursts, as it will be explained later.

As it has been outlined in the background section, one technical problem present in the field is how to provide miniaturized implantable intravascular pressure sensors enabling minimal invasiveness. For instance, the possibility of percutaneous deployment (by injection or catheterization) is an important advantage of this system, instead of requiring a surgical procedure. To avoid the need of batteries, wireless power transfer (WPT) by volume conduction (also known as galvanic coupling) is a suitable alternative for powering said pressure sensors, and at the same time, for enabling intrabody communications and communications with external devices.

In a first inventive aspect, the invention refers to a pressure sensor, adapted to be implanted inside a human or animal vessel (i.e., a vein, artery, or lymphatic vessel) for measuring intravacular pressure, comprising:

- A hermetic capsule comprising at least a partially flexible portion. Said portion enables pressure transmission from the capsule outside to its inside.
- An electronic circuit housed in the capsule, said electronic circuit being adapted to generate an electrical transduction pressure signal resulting from a deformation of the partially flexible portion of the capsule (either by direct transduction or by pressure transmission to a fluid inside the capsule), such that the transduction signal is dependent on the absolute pressure outside the capsule.
- An electrode pair electrically connected to the electronic circuit, wherein each electrode of the electrode pair is, at least in part, arranged externally to the capsule.

Advantageously, the electronic circuit of said pressure sensor comprises:

- A digital control unit (DCU). The DCU would refer, without exclusion, to a computer processing unit (CPU), a field-programmable gate array (FPGA) or equivalent general digital microprocessor equipped with processing means to record and process the signals collected by the pressure sensor.
- An interrogation stage adapted to receive an interrogation signal transmitted by volume conduction to the electrode pair; to process said interrogation signal by means of the digital control unit; to generate, in response to the interrogation signal, an electrical transduction pressure signal; and to transmit, in response to the interrogation signal, the electrical transduction pressure signal to a medium by volume conduction, through the electrode pair.
- A power transferring stage adapted to receive at least part of the interrogation signal transmitted by volume conduction to the electrode pair, and to transfer the power associated to said at least part of the interrogation signal to the digital control unit and to the interrogation stage.

In this way, in the present invention the electronic circuit of the pressure sensor is active, and digital, and it is not only interrogated by WPT but also it is powered by WPT based on volume conduction. The use of digital electronics allows implementing volume-conduction interrogating and powering functionalities under a single electronic architecture.

In particular embodiments of the invention, the capsule body of the pressure sensor is made, at least in part, of metallic material. Preferably, this material is a biocompatible metal, such as titanium or stainless steel, adequate for permanent implantation in an animal or human body.

In order to achieve that the hermetic capsule (or at least a portion of it) exhibits some degree of flexibility, the capsule walls must be thin (preferably, a thickness lower than 0.5 mm). Alternatively, the capsule (3) body geometry may facilitate the flexion of the capsule, for instance, by implementing corrugated walls or by using capsules with elongated cross sections, such as rectangular or oval cross sections. In this sense, in more preferred embodiments of the invention, the capsule exhibits an ellipsoidal or tubular shape.

The electromechanical architecture of the hermetic capsule also plays a relevant role in the field of active implantable medical devices (AIMDs). In the context of the invention, two main alternatives can be used for this architecture:

- Alternative 1: integrating an absolute pressure sensor within the electronics (e.g., a microelectromechanical (MEMS) sensor, and to develop a capsule that allows pressure transmission between the external medium and an internal medium filling the capsule, like oil.

- Alternative 2: Arranging the semi-flexible capsule in such as a way that it senses the absolute pressure. Preferably, a capsule made of a biocompatible metal (e.g., titanium) is a convenient option, as it allows sensing the pressure by measuring capacitance (or other transduction mechanisms) between a capsule wall and an internal conductor inside the electronic circuit. Such solution, where the use of a hermetic metallic capsule for capacitive pressure transduction, is particularized for the case of a capsule containing an active electronic circuit connected via feedthroughs with an external electrode pair.

The first of the above alternatives would lead to pressure sensors of larger dimensions in comparison with the second alternative, but it is easier to manufacture. These two alternative designs for the capsule, without limitation to other embodiments covered by the scope of the claims, are described as follows.

In some embodiments of the invention, the pressure sensor further comprises a pressure transmission fluid housed inside the capsule, wherein the fluid is arranged between the flexible portion of the capsule and the electronic circuit, and wherein the electrical circuit further comprises a pressure transducer (e.g., a capacitive or piezoresistive MEMS sensor). Preferably, the fluid is non-compressible (e.g., silicone oil) and serves for maximizing pressure coupling. Other alternatives may use compressible fluid (e.g., air) contained within the capsule. In this way, this embodiment corresponds to the aforementioned first alternative.

In other advantageous embodiments of the pressure sensor, the partially flexible portion of the capsule is electrically connected to the electronic circuit and capacitively coupled to said electronic circuit through a conductor, forming a pressure transducer whose capacitance depends on the deformation of the partially flexible portion of the capsule. Advantageously, in this embodiment the capsule is adapted to work as the diaphragm of the pressure sensing mechanism. In this way, this embodiment corresponds to a possible implementation of the aforementioned second alternative.

In additional embodiments of the pressure sensor, said sensor comprises two conductors and the electronic circuit is capacitively coupled to the capsule through said conductors, forming two pressure-dependent capacitive elements (transducers) connected in series. Advantageously, in this embodiment the capsule is also adapted to work as the diaphragm of the pressure sensing mechanism. In this way, this embodiment corresponds to a possible implementation of the aforementioned second alternative.

In further preferred embodiments of the invention, the pressure sensor comprises fixation means adapted for attaching it to a vessel. Preferably, the fixation means comprise the electrode pair. In even further embodiments, the electrode pair or the fixation means comprise at least one of the following: a stent structure, a cable structure (which serves only for the electrodes, not for fixation purposes), or a wire loop structure. Preferably, the fixation means comprise flexible structures adapted to anchor the pressure sensor to the vessel. In certain embodiments, the fixation means (e.g., a portion of a stent) can be further used as the electrode pair.

In certain embodiments, the fixation means of the pressure sensor comprise at least partially non-insulated flexible wire loops, or even said wire loops are completely uninsulated.

In accordance with a further preferred embodiment of the invention, the exterior of the capsule is coated with a thin and flexible layer of dielectric material (e.g., parylene). In the case of metallic capsules, by preventing short circuiting of the electrode pair, this insulator maximizes power transfer by volume conduction. Advantageously, the insulating layer minimizes interferences during the pressure measurement process caused by the high frequency current bursts delivered by the interrogation units or caused by physiological biopotentials. Besides its dielectric properties, this coating may be also used to provide some other functional features such as dry lubricity, which may be advantageous during the deployment of the pressure sensor by minimally invasive means. In this way, this insulating layer provides enhanced biocompatibility (e.g., for minimizing thrombogenesis) and/or additional therapeutic functionalities (e.g., sustained release of embedded therapeutic drugs for blocking cell proliferation and thus preventing in-stent restenosis).

In a more preferred embodiment of the invention, the external electrodes are also coated with a very thin layer of insulating material (e.g., parylene). Again, this coating may provide some advantageous features such as dry lubricity, enhanced biocompatibility, or therapeutic functionalities. However, in this case, if the material is a dielectric, the coating will be disadvantageous for volume conduction. Therefore, the thickness of the dielectric coating will have to be of less than 1 micrometre to ensure that power transfer by volume conduction at high frequency is effective. Alternatively, if a thick coating of dielectric material is required, or if low frequency or dc currents must be able to flow through the pressure sensor, openings can be created in the dielectric coating by different methods (e.g., by laser ablation).

In certain embodiments, the power transferring stage of the electronic circuit comprises a blocking capacitor connected in series with the electrode pair, adapted so as to prevent passage of direct current (dc) and to enable the flow of high frequency current to and from the electronic circuit. The blocking capacitor is typically required to prevent the passage of very low frequency currents, essentially dc, that would cause electrochemical reactions at the electrode pair and could damage both the surrounding living tissues and the electrodes themselves. In said embodiments, the interrogation stage comprises a demodulator unit adapted to compare low pass filtered signals obtained with a rectifier and a digital converter connected to a pressure sensor, either a sensing capacitor or a piezoresistive sensor. Furthermore, in these embodiments the digital control unit is adapted to generate an electrical signal corresponding to the pressure measurement by modulating the load of the pressure sensor.

In more preferred embodiments, the blocking capacitor does not need to have a large capacitance value (hundreds of nanofarads, or even microfarads). Instead, in these preferred embodiments, it is enough arranging a capacitor, preferably in the order of tens of nanofarads (or even less), which is dimensioned for allowing the flow of high frequency currents (higher than 1 MHz). Advantageously, the lower capacitance value of the blocking capacitor is due to the fact that the pressure sensor does not perform stimulation. Moreover, this enables scaling down the size of the capacitor and further favours the miniaturization of the pressure sensor capsule.

A second object of the invention refers to a system comprising one or more pressure sensors as previously described, along with a reading unit which in turn comprises:

- A signal generator adapted to generate an interrogation signal; and to transmit said interrogation signal to the pressure sensors through a medium (e.g., a biological fluid, like blood) by volume conduction.
- Receiving means adapted to receive an electrical signal corresponding to the pressure measurement from the pressure sensor through the medium by volume conduction.
- Processing means adapted to process information associated to the electrical signal corresponding to the pressure measurement.

Optionally, the reading unit may also include a display or other graphical representation means to show information to a user.

Preferably, the reading unit is a battery powered hand-help unit, embodied as an external device. Alternatively, the reading unit is formed by two parts, namely: an implantable part for reading an implant inside a body, and an external part wirelessly communicated with the implantable part. In particular, the implantable part can consist of a subcutaneously implanted sub-unit capable of generating current bursts and also for taking measurements. In turn, the external part is capable of processing data wirelessly transmitted by the implanted part, and for representing the measurements for example in a display and for generating alarms, and further transmitting the measurements to a third device (e.g., another computer, a smartphone, a server, etc.) comprising storage means suitable for saving historical data record of pressure measurements and associated information (time at which the measure is taken, etc.).

Advantageously, multiple pressure sensors (also referred to as implants) can be independently interrogated by the reading unit because they can be addressed individually.

In preferred embodiments of the system, the reading unit is adapted to be implanted inside a human or animal body. More preferably, in these embodiments the processing means are further adapted to wirelessly transmit the pressure measurement to an external device. Alternatively, the reading unit electrically is placed out of the body. The reading unit is preferably powered by batteries.

Optionally, the reading unit is adapted to deliver bursts or an alternating current at a carrier frequency 1-100 MHz, with burst duration between 0.1 $\mu$s and 10 ms, and a repetition rate between 0-100 kHz. More particularly, delivering the bursts at a frequency 1-20 MHz is particularly advantageous. In a further preferred embodiment, the bursts delivered by the reading unit are sinusoidal.

A third object of the invention refers to an interrogation and powering method for operating the system described above in order to obtain pressure measurements, characterised in that it comprises the realization of the following steps:

- Generating, with the reading unit, an interrogation signal and transmitting said interrogation signal to at least one pressure sensor through a medium, by volume conduction.
- Receiving the interrogation signal in the pressure sensor.
- Transferring power associated to at least part of the interrogation signal to the digital control unit and to the interrogation stage of the electronic circuit of the pressure sensor.
- Processing said interrogation signal by means of the digital control unit of the electronic circuit of the pressure sensor.
- Generating, in response to the interrogation signal, an electrical signal corresponding to the pressure measurement resulting from the deformation of the partially flexible portion of the capsule, by means of the interrogation stage of the electronic circuit of the pressure sensor.
- Transmitting the pressure measurement to the reading unit through the medium by volume conduction, by means of the interrogation stage of the electronic circuit of the pressure sensor.

In preferred embodiments of the previous method, the interrogation signal generated by the reading unit is a high-frequency alternating current comprising one or more bursts which encode the address of at least one pressure sensor. Said current reaches the at least one pressure sensor by volume conduction through the medium.

In further preferred embodiments of the method, the step of transmitting the pressure measurement comprises modulating the load that the at least one pressure sensor exhibits to the passage of high-frequency current flowing through the medium by volume conduction.

In general terms, the pressure sensor and the system comprising it provide the following additional advantages in comparison with known alternatives in the field:

- Less cumbersome interrogation (external) systems. This invention provides a system in which the reading unit can be also implanted in the body.
- Possibility to perform continuous measurements or automatic measurements at regular intervals.
- Easily enable measurements from multiple nearby sensors because of the communication capabilities it could have. For instance, the interrogation signal itself encodes the address of the pressure sensor to which it is sent, either a physical or a logical address (i.e., MAC or IP address). This provides a selective mechanism of interrogating and powering different sensors with the same reading unit. More preferably, the communication and addressing protocols can be customized to minimize the duration of the communications, as well as reducing burst duration and power loss involved in said communications.
- The fixation elements (e.g., wire loops) are not necessarily insulated, which eases the manufacturing.

In the scope of the invention, the following definitions must be considered about certain technical concepts. Throughout the description, digital electronics and active electronics are treated as synonyms. High frequency refers to frequencies above 1 MHz, and more preferably, in the range 1-100 MHz. The medium preferably corresponds to a biological fluid (e.g., blood), flowing within a vessel (e.g., an artery or vein). Moreover, the pressure sensor is also referred to as implant in the description. Finally, the term intravascular pressure refers to the pressure of any intrabody cavity or vessel, i.e., blood or lymphatic vessels.

## BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:

Figure 1 shows a schematic representation of the main elements of a particular embodiment of the sensor, which comprises partially insulated electrodes (anchors).

Figure 2 illustrates four different configurations of the pressure sensor of the invention, attached to a vascular stent structure, a cable structure and/or wire loops.

Figure 3 illustrates a possible implementation of the intravascular pressure sensing implant according to the invention. In this case the fixation loops are completely uninsulated, unlike those ones from Figure 1.

Figure 4 corresponds to a schematic representation of the preferred sensor for pressure transduction, comprising at least a flexible portion in the capsule and capacitive pressure detection.

Figure 5 shows a pressure sensor wherein the capsule can be connected directly to the electronics, thus forming a single capacitor.

Figure 6 shows another sensor wherein the capsule comprises a conductor.

Figure 7 shows a pressure sensor with the capsule filled with fluid.

Figure 8 displays a schematic representation of a preferred capsule shape, which is ellipsoidal.

Figure 9 shows an alternative design for the hermetic capsule.

Figure 10 displays the general architecture of the electronics of the implant (pressure sensor).

Figure 11 illustrates an embodiment according to the system of this invention for monitorization of restenosis.

In these figures, the following reference numbers are used:

| | |
|---|---|
| 1, 1' | Pressure sensors |
| 1" | MEMS pressure transducer |
| 2 | Vessel |
| 3 | Capsule |
| 3' | Flexible portion of the capsule |
| 3" | Dielectric coating of the capsule |
| 4 | Electronic circuit (digital circuit) |
| 4' | Substrate for the electronic circuit |
| 5, 5' | Electrode pair of the pressure sensor |
| 6 | Reading unit |
| 7 | Battery or powering system of the reading unit |
| 8, 8' | Skin electrodes |
| 9 | Graphical representation means (display) of the reading unit |
| 10 | Signal generator of the reading unit |
| 11 | Wire loop structure |
| 12 | Fixation means |
| 13 | Inner part of the electrode pair |
| 14 | Outer part of the electrode pair |
| 15 | Medium (blood, tissue, etc.) |
| 16, 16', 16" | Stent structure |
| 17 | Cable structure |
| 18 | Feedthroughs |
| 19, 19' | Conductors for pressure measurement |
| 19" | Conductor for connecting the capsule and the electronic circuit |
| 20 | Receiving means |
| 21 | Processing means |
| 22 | Rectifier |
| 23 | Power transfer stage of the electronic circuit |
| 24 | Blocking capacitor |
| 25 | Interrogation stage |
| 26 | Demodulator unit |
| 27 | Digital converter |
| 28 | Sensing capacitor |
| 29 | Digital control unit |
| 30 | Load |
| 31 | Voltage regulator |

## DETAILED DESCRIPTION OF THE INVENTION

Different possible configurations of the pressure sensor, the system and the method will be now described for illustrative purposes.

As disclosed in the preceding sections, the invention refers to an implantable intravascular pressure sensor (1) for measuring pressure due to the presence or flow of a fluid, such as blood, in the vessel (2). A major advantage of the pressure sensor (1) is that it does not require a bulky system for feeding its electronics and, thereby, the miniaturization and the operational life of the implantable device is not limited by this feature. To achieve this, volume conduction (also known as galvanic coupling) is used as a wireless power transfer (WPT) mechanism for the sensor (1), and active (digital) electronics is required. The pressure sensors (1) obtained with this technology based on volume conduction are much thinner (< 0.5 mm), therefore less invasive than other implant technologies. Advantageously, this provides further miniaturization of the whole pressure sensor (1) is required to avoid surgical procedures for its deployment within the vessel (2).

The structure of the sensor (1) includes a hermetic capsule (3) comprising at least a partially flexible portion, which enables pressure transmission from the outside to the inside of said capsule (3), and an electronic circuit (4) housed in the capsule (3), adapted to measure a pressure signal resulting in the vessel (2). The hermetic capsule (3) also serves as an external housing for the sensor (1), thereby protecting the electronic circuit (4). The sensor (1) further comprises an electrode pair (5, 5') electrically coupled to the electronic circuit (4) and passing through the capsule (3), such that each electrode of the electrode pair (5, 5') is, at least in part, arranged externally to the capsule (3) and adapted to receive power by volume conduction through a medium, such as the blood or muscle tissues. Moreover, in a preferred embodiment of the invention, at least one electrode of the electrode pair (5, 5') or a structure attached to the capsule (3) is preferably a flexible structure configured for anchoring the implant to a vessel (2), for instance, an artery or vein.

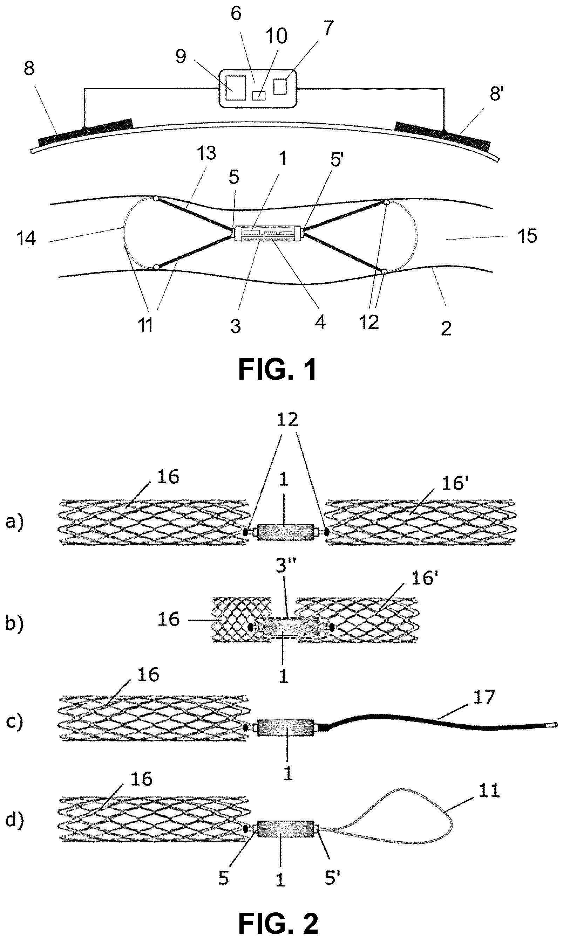

To obtain a pressure measurement, the pressure sensor (1) is preferably used in combination with a reading unit (6), which is adapted for interrogating and powering said pressure sensor (1). In this way, as shown in Figure 1, one or more pressure sensors (1) deployed within human or animal vessels (2) and a reading unit (6) form the system according to the invention. It is worth remarking that the reading unit (6) may be external or also implanted (e.g., subcutaneously). The reading unit (6) for interrogating the implant is preferably powered with a battery (7) and comprises skin electrodes (8) adapted for their placement on a skin portion, close to the implant or implants to be interrogated, and a display (9) configured for the visualization of the measurement values obtained. Further, the reading unit (6) preferably comprises a signal generator (10), which will be later described in greater detail. The reading unit (6) is shaped as a pod and it is fixed on the skin over the location of the implant, for instance, by a fastener or sticking plaster.

The reading unit (6) stores measurements in a memory means or transmits those measurements by radio communication to a nearby computerized device, such as a smartphone. Advantageously in the invention, volume conduction is suitable for powering the implants from the reading unit (6) and for enabling bidirectional digital communications between the pressure sensor (1) and said reading unit (6). In particular, in order to meet the requirements of safety standards and to be able to operate deep implants, the use of volume conducted bursts of alternating (ac) currents at frequencies in the range from 1 MHz to 100 MHz is preferable. In this way, the use of volume conduction is crucial for the invention as it avoids the need of any bulky component within the pressure sensor (1) for generating power or for receiving power sent by the reading unit (6).

The reading unit (6) can produce measurements of pressure by processing either the signals emitted by the sensor during the interrogation signal or after it. The reading unit (6) can also combine measurements acquired during and after the interrogation signal in order to improve the accuracy of the pressure measurement.

As shown in the embodiment of Figure 1, the electrode pair (5, 5') is preferably conformed as flexible metallic wire loop structures (11), wherein a portion of said wire loop structures (11) further serves as fixation means (12) for attaching the pressure sensor (1) to a vessel (2). Advantageously, this configuration enables using a portion of the electrode pair (5, 5') as fixation means (12). Optionally, in the embodiment displayed in Figure 1, an inner part (13) of the wire loop structures (11) is covered with a layer of an insulating material (preferably parylene), while an outer part (14) is exposed to a medium (15), (e.g., flowing blood). This configuration for the electrodes facilitates implant deployment, anchorage, and extraction. For deployment, the wire loop structures (11) can be readily folded within a catheter for enabling minimally invasive implantation through catherization. After deployment, the wire loop structures (11) will unfold due to their flexibility or because of shape memory if the wire is made of a metal exhibiting such property such as nitinol and, by pressing the vessel (2) walls, will anchor the implant within the vessel (2). Advantageously, this embodiment enables a minimally invasive implant extraction as the pressure sensor (1) can be extracted with a catheter by pulling out with a hook one of the two wire loop structures (11). Alternative embodiments of the invention do not require any insulation of the electrodes.

Figure 2 shows different preferred embodiments of the fixation means (12) and the electrode pair (5, 5') of the pressure sensor (1). As illustrated in Figure 2a, the electrode pair (5, 5') can also consist in segments of an intravascular stent structure (16, 16'). In Figure 2a the segments are displayed as symmetric (of substantially similar dimensions) and separated by the capsule (3) of the pressure sensor (1). On the other hand, as shown in Figure 2b, the segments can be nonsymmetric (i.e., one longer than the other) and they can be brought closer (for instance, over the capsule (3)) provided that they are not short circuited by direct contact or by the capsule (3). Also, Figure 2b comprises a dielectric coating (3") on the capsule (3) to prevent short circuiting the two segments of the stent structure (16, 16'). Figure 2c displays another possible configuration, in which one of the electrodes of the electrode pair (5, 5') is a portion of a stent structure (16), while the other electrode comprises a cable structure (17) that does not serve as fixation means for the sensor (1). In this way, thanks to the cable structure (17), one of the electrodes of the electrode pair (5, 5') is wired at some distance from the capsule (3). This embodiment may be useful in scenarios where the dual stent structure (16, 16') from Figures 2a or Figure 2b cannot be made sufficiently long to pick up the necessary voltage difference for operation of the electronic circuit (4). Figure 2d corresponds to another configuration, in which one of the electrodes of the electrode pair (5, 5') is a portion of a stent structure (16) while the other electrode is attached to a wire loop structure (11). A portion of said wire loop structure (11) may serve as fixation means (12). This embodiment may be useful in scenarios where the dual stent structure (16, 16'), see Figures 2a-2b, cannot be made sufficiently long and the presence of a loose wire (as in Figure 2c) is not appropriate.

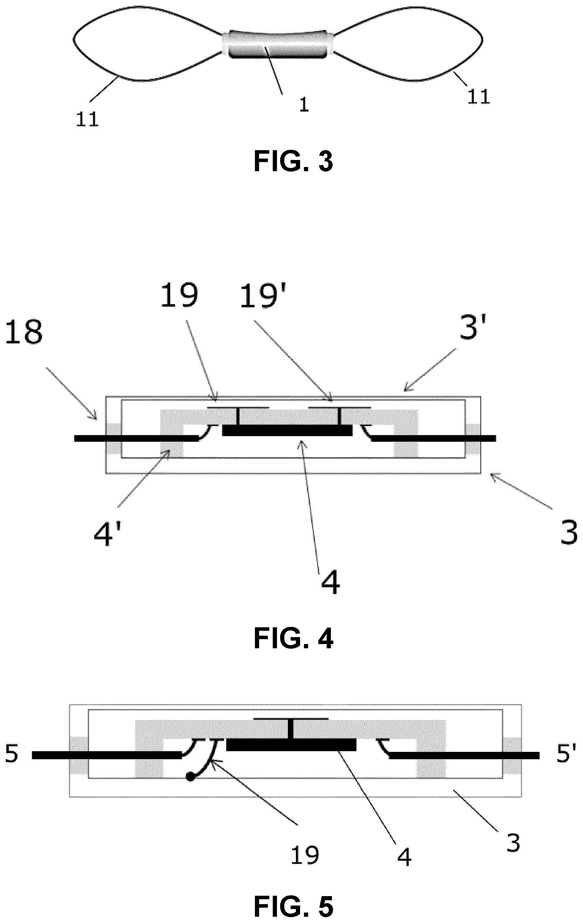

A further preferred embodiment of the invention is shown in Figure 3, corresponding to the case in which the fixation wire loops structures (10) of the intravascular pressure sensor (1) are completely uninsulated. In this way, two fixation wire loops structures (10) (or alternatively, stent segments) are attached to both ends of a compliant metallic hermetic capsule (3) having a diameter lower than 1 mm and which further includes a digital electronic circuit (4).

An optional aspect of the invention is taking advantage of the flexible and hermetic capsule (3) to implement the mechanism of pressure transduction, as displayed in the preferred embodiment of Figure 4. Advantageously, the use of the capsule (3), along with specific design of its shape (e.g., tubular, or ellipsoidal body) enables to protect the electronic circuit (4) which is placed over a substrate (4') (e.g., ceramic) for the electronics. At the same time, the capsule (3) acts as a constituent part of the pressure sensor (1). In this case, the capsule (3) is metallic (e.g., titanium or other biocompatible material) and comprises thin walls with feedthroughs (18) for each electrode of the electrode pair materials (5, 5'), being said feedthroughs (18) placed at opposite ends in the longitudinal direction of the pressure sensor (1), as displayed in Figure 4. The absolute pressure of the medium (15) outside the capsule (e.g., the blood flow) is transduced into flexible deformation of the capsule (3) which will be in turn transduced into capacitance changes between the conductive portion of the capsule and two parallel conductors (19, 19') placed inside the capsule (3) and located beneath the flexible portion (3'), for instance, on an integrated electronic circuit (4). Preferably, as illustrated in Figure 4, two in series capacitors are formed between the conductors (19, 19') of the implant and the capsule (3) wall. The connection between the electrode pair (5, 5') and the electronic circuit (4) can be performed through hermetic feedthroughs (18) to ensure the hermeticity of the capsule (3). Note that in the embodiment of Figure 4, the flexible portion (3') of the capsule (3) (or a mechanical element attached to it) must be conductive (metallic).

Alternatively, the capsule (3) can be connected directly to the electronics thus forming a single capacitance. This embodiment is illustrated in Figure 5, wherein one conductor (19) is connected to the electronic circuit (4) and is located beneath the flexible portion (3'). In this way, an electrical connection is stablished between the electronic circuit (4) and the capsule (3) wall, thus creating a pressure-dependent capacitance.

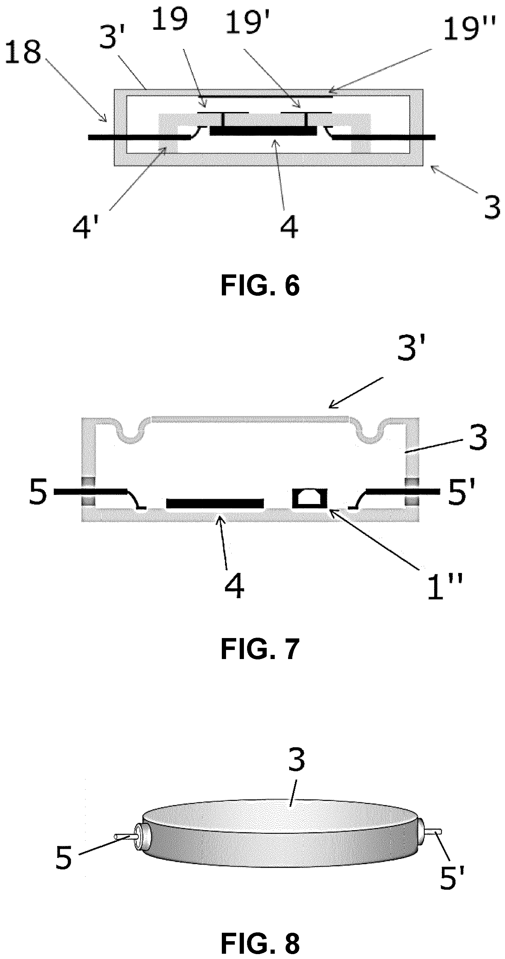

Another optional embodiment is a variant of the one illustrated in Figure 4 but comprising a conductor (19") in the flexible portion (3') of the capsule (3), and wherein the capsule is made of ceramic or glass instead of a metallic material. This embodiment is shown in Figure 6.

Other preferred embodiments of the pressure sensor (1) comprise one or more piezoresistive elements (e.g., strain gauges) that are attached (e.g., glued) to the inner wall of the flexible portion (3') of the capsule (3) thus forming a resistive pressure sensor which is connected with conductors (19, 19') (e.g., wires) to the electronic circuit (4).

An alternative for pressure transduction within the embodiments of the invention is to include a conventional MEMS pressure transducer (1") integrated in the electronics of the implant (e.g., in the same printed circuit board or in the same integrated circuit) and wherein the external pressure surrounding the capsule (3) is transmitted to the pressure sensor via one of the following possibilities:

a) Via a non-compressible fluid (e.g., silicone oil) contained in the capsule (3), as illustrated in Figure 7. In this case, the flexible portion (3') of the capsule (3) only needs to flex slightly to effectively transmit the pressure difference between the outside of the capsule (3) and the inside. Silicone oil is preferred due to its biocompatibility, which ensures safety in case of leakage. Besides, it exhibits compatibility with electronic components. The flexibility of the portion (3') of the capsule (3) can be enhanced by corrugation.

b) Via a compressible fluid, like air, contained within the capsule (3). In this case, the hermetic capsule (3) entirely, or partially, must exhibit a large degree of flexibility or mechanical compliance so that the pressure is effectively transmitted from the exterior of the capsule (3) to the interior.

The conventional MEMS pressure transducer (1") can be, for example, a MEMS piezoresistive or capacitive pressure sensor.

The capsule (3) body may have an elliptical disk shape, as illustrated in Figure 8. Advantageously, this embodiment is based on two major features:

1) The top or the bottom capsule (3) walls can substantially deflect under pressure and thus facilitating pressure sensing (higher sensitivity for the capacitive pressure transduction).

2) The capsule (3) body opposes low fluid (hydrodynamic) resistance and prevents, or minimizes, stagnation regions where blood thrombi can develop.

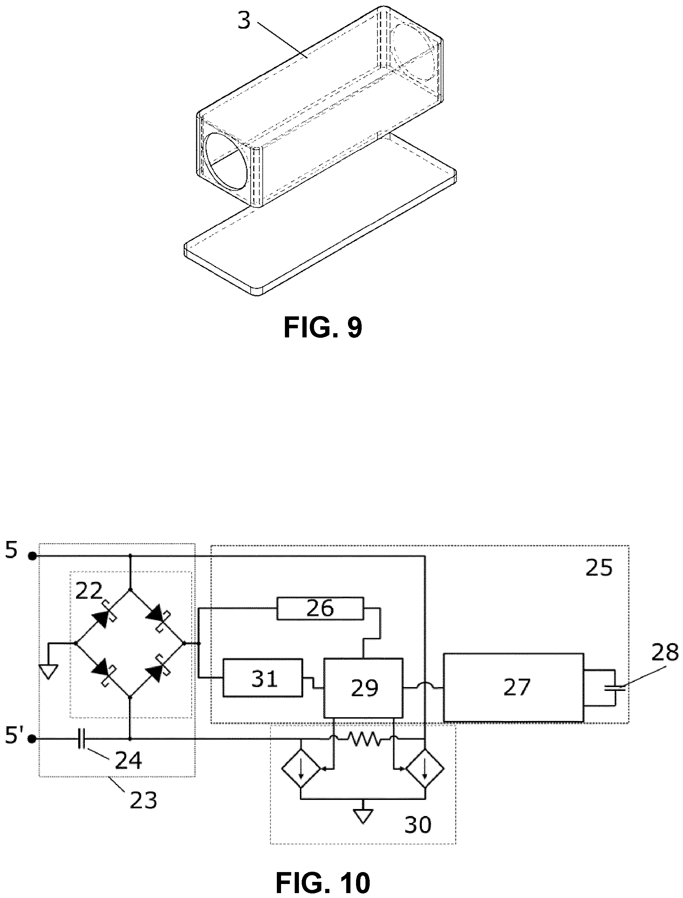

Figure 9 shows another possible design of the hermetic metallic capsule (3) for housing the electronic circuit (4) of the implant. Said capsule (3) acts as the diaphragm of the pressure sensing mechanism: the capsule (3) wall itself is used for sensing blood pressure changes.

An essential feature of the invention relates to how the wireless power transfer takes place. The obtained dc power is maximized by delivering interrogation signal (which is an ac field) in the form of short bursts rather than continuously. The maximum attainable dc power depends on the conductivity of the medium (15), e.g., tissue or a body fluid. Such dependency is minimized by delivering the ac field in the form of short bursts rather than continuously, which enhances the wireless power transfer.

The fundamental structure of the powering and interrogation method comprises the following steps:

1. The reading unit (6), via a signal generator (10), generates an interrogation signal, preferably comprising bursts of high-frequency alternating current - not necessarily sinusoidal - that reach one or more pressure sensors (1) by volume conduction. The interrogation signal includes the address of a specific sensor. In this way, even if the interrogation signal may reach several pressure sensors (1), preferably only the sensor (1) corresponding to the address would be read by the reading unit (6).

2. By means of the electrode pair (5, 5'), the pressure sensor (1) picks up a portion of the high frequency current flowing through tissues and by internal rectification and regulation produces a dc voltage adequate to power its electronics.

3. During the burst or afterwards, the electronic circuit (4) of the pressure sensor (1) performs a pressure measurement which is digitally codified.

4. During a burst, the pressure sensor (1) transmits the measurement by modulating the load it represents to the passage of high frequency currents (alternatively the pressure sensor (1) transmits the measurement by delivering voltage signals across its electrodes after the burst). We recall that at high frequencies (in the order of MHz), living tissues can be approximated to behave as resistances and the electrode interface impedances can be neglected.

5. The receiving means (20) of the reading unit (6) detects the electrical transduction pressure signal. Then, the processing means (21) demodulate and process the said signal to produce a measurement which is either stored in memory or sent to another device by conventional means (e.g., radio communication). Optionally, the pressure measurement is shown in a display (9) or screen included in the reading unit (1).

Both the absolute maximum attainable power and the maximum attainable dc power exhibit a distinctive maximum for a specific load resistance (i.e., optimum load resistance). If the ac field is delivered in the form of bursts rather than continuously, it is possible to set the value of the optimum load resistance by adjusting the duty cycle of the bursts.

Figure 10 illustrates a basic electronic circuit (4) topology for the pressure sensor (1) of the invention, based on capacitive sensing. The electrode pair (5, 5') pick up a part of the high-frequency current of the interrogation signal for powering the circuitry through the diode bridge full-wave rectifier (22). Then, the digital electronic circuit (4) comprises:

- A power transfer stage (23) including a diode bridge full-wave rectifier (22) and a blocking capacitor (24) connected in series with the electrode pair (5, 5'), adapted so as to prevent passage of dc current and to enable the flow of high frequency current to and from the electronic circuit (4). In this way, this electronic circuit (4) performs the rectification of high-frequency current bursts. A capacitor is used to power the electronics of the sensor in-between high frequency current bursts.

- An interrogation stage (25), which in turn includes:

a) An amplitude demodulator unit (26) that compares low-pass filtered signals obtained with the rectifier (22) and a digital converter (27) - particularly a capacitance-to-digital converter (further abbreviated as CDC)-, which is connected to a sensing capacitor (28). A demodulator unit (26) extracts information about the direction of the pressure sensor (1) to be interrogated. In this case, the demodulator unit (26) uses an amplitude-shift key (ASK), which allows a simpler demodulator unit (26) architecture. Alternatively, other digital modulation schemes like frequency-shift key (FSK), phase-shift key (PSK) or Quadrature Phase-Shift Key (QPSK) are also possible.

b) A sensing capacitor (28) connected to the digital converter (27).

- A digital control unit (29), for instance, a FPGA which is adapted to generate an electrical signal corresponding to the pressure measurement by modulating the load (30) of the pressure sensor (1). Said load (30) corresponds to the impedance across the electrode pair (5, 5'), that is, the impedance of the medium (15). This impedance is determined by the passive electrical properties of the medium (15), i.e., the living tissues, and by the geometry of the pressure sensor (1) and its electrodes (5, 5'). In some cases, it is acceptable to model it as a resistance.

Different circuit topologies are known that can be used to implement the CDC, which provides a digital value proportional to the capacitance value of a capacitor. These CDC architectures are often classified by the type of Analog-to-Digital Converter (ADC) used internally. Examples of such CDC architectures are the Sigma Delta ($\Sigma\Delta$) CDCs, the Successive Approximations (SAR) CDCs or the double slope CDC. Another relevant group of CDC architecture converts the capacitance value into a time varying signal and the measurement of the timing parameters are then converted to digital domain. Examples of such time-based CDC architectures are the Pulse Width Modulation. Any of the aforementioned CDCs, or equivalent ones, can be used in the context of this invention. Optionally, the electronic circuit (4) further includes a voltage regulator (31) to obtain a suitable voltage to feed the DCU (25).

Alternatively, other implementations of the electronic circuit (4) may use piezoresistive sensing of pressure variations instead of capacitance changes. For instance, in the circuit from Figure 10, the set of sensing capacitor (28) and CDC can be replaced by a piezoresistive pressure transducer.

To obtain an efficient wireless power transfer, the parameters of the interrogation signal (frequency, burst rate, burst duration, etc.) must be properly established. Preferably, the pressure sensor (1) electronics operates with a burst frequency below 100 MHz. In more preferred embodiments, the burst frequency range is 1-20 MHz. Advantageously, this range provides a more isotropic behaviour of the tissues (e.g., muscle tissues can be considered isotropic above 1 MHz) and, at the same time, minimizes the skin effect.

The injected alternating currents for interrogation, which are either current controlled or voltage controlled, are specially selected to be innocuous to the body. This is accomplished by ensuring that these currents are of sufficient frequency to prevent unsought stimulation of excitable tissues (first requirement), and their power is low enough to prevent excessive heating of tissues due to Joule heating (second requirement).

Furthermore, it is required that the interrogation signal encode the address of the pressure sensor (1) to be reached (e.g., a physical (MAC) or a logical (IP) address). More preferably, the bursts encode the target pressure sensor (1) at which the interrogation signal is sent.by using known techniques of digital addressing, thereby enabling a selective interrogation. This is particularly convenient when multiple pressure sensors (1) are read, excited (interrogated) and powered by the same reading unit (1) or said sensors (1) are implanted close to each other.

The first requirement can be readily met by using currents whose power spectral density is well above 100 kHz. For instance, currents with a frequency (f) above 1 MHz, are desired.

Furthermore, frequencies below 100 MHz are preferred, to prevent that the skin effect becomes significant, and the operation of implants at deep locations is hindered.

The second requirement is achieved by delivering short bursts. For a given voltage gradient (E) required to ensure operation of the implants (e.g., 200 V/m), burst duration (B) and burst repetition frequency (F), are selected to ensure that power dissipation in the tissue where the implants are located does not reach a safety threshold.

Safety thresholds for electromagnetic power dissipation in tissues are usually specified by the so-called Specific Absorption Rate (SAR), which is measured in W/kg. SAR can be calculated with the following expression:

$$SAR = \sigma(E_{RMS})^2/\rho$$

where $\sigma$ is the electrical conductivity of the tissue (S/m), $p$ is the mass density of the tissue (kg/m$^3$) and $E_{RMS}$ is the root mean square value of the electric field in the tissue (V/m).

From that expression it can be obtained the following requirement for F and B in the case of sinusoidal bursts:

$$FB < 2\rho SAR_{MAX}/\sigma E^2$$

A SAR value of 2 W/kg is considered to be a safe threshold in all circumstances according to different standards. If the above expression is particularized for the case of muscle tissue at 10 MHz ($\sigma_{10\ MHz} = 0.62$ S/m, $\rho = 1060$ kg/m$^3$) and it is assumed that the field required for operation is 200 V/m, the 2 W/kg limit yields FB < 0.17 s/s. Thus, for instance, if the burst duration, is 10 $\mu$s, the maximum repetition frequency would be 17 kHz. If the above expression is particularized for the case of blood at 6.67 MHz, which is the central frequency of an ISM (Industrial, Scientific and Medical) band and hence it is a suitable frequency to electromagnetic compatibility issues, ($\sigma_{6.67\ MHz}$ = 1.066 S/m, $\rho$ = 1060 kg/m$^3$) and it is assumed that the field required for operation is 200 V/m, the 2 W/kg limit yields FB < 0.099 s/s. Thus, for instance, if the burst duration, is 10 $\mu$s, the maximum repetition frequency would be 9.9 kHz.

It must be noted that the SAR limitation must not only be met where the implants are located but also in all tissue regions where the interrogation currents flow through. Therefore, since current densities (and voltage gradients) will be probably higher in the vicinity of the current injecting electrodes of the reading unit, the FB product will have to be scaled down.

The interrogation signal may consist of a modulated sinusoidal waveform with a carrier frequency between 100 kHz and 100 MHz which is delivered as bursts with a duration between 0.1 $\mu$s and 10 ms, and a repetition frequency between 0 Hz (i.e., single burst interrogation) and 100 kHz.

The digital modulation scheme employed in the communications may guarantee that throughout the whole transmission of the interrogation signal, power is delivered to the pressure sensor (1). Particularly, even in the case of a long burst transmission containing only 0 values, it would be desirable that a high-frequency current is transferred to the pressure sensor (1) in order to feed the electronic circuit (4). For addressing this case, the use of Manchester codification is convenient as it provides a constant average amplitude of the signal, which is required by the amplitude demodulator unit (26), as well as self-clocking capabilities. Alternatively, the communication can comprise only a fraction of the high frequency burst and during said fraction, the powering of the electronic circuit (4) can be kept by means of a capacitive element associated with power regulator as it is commonly performed in voltage regulators.

A preferred use is the detection and monitorization of in-stent restenosis When in-stent restenosis occurs, that is, when excess tissue or plaque builds up on the inner walls of the stent, blood flow is obstructed thus changing the pressure distribution along the stent. An alteration of local pressure, which can be detected with the system of the invention, may be indicative of restenosis. In general, upon the onset of restenosis, the average pressure during the cardiac cycle will increase pressure upstream the stent and will decrease downstream.

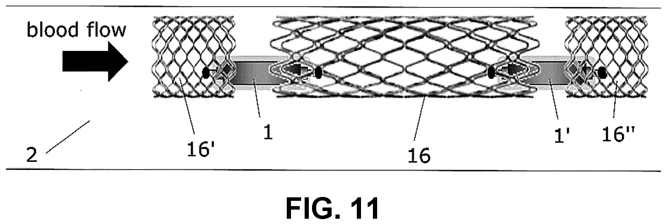

In an optional system, a single pressure sensor (1), which can be located upstream in the stent, suffices to detect an abnormality in blood flow and thus warns about a possible complication. However, in another preferred embodiment of the system of the invention, two pressure sensors (1, 1') are embedded in a stent structure (16, 16', 16"), one sensor (1) upstream and another sensor (1') downstream, as displayed in Figure 11. By obtaining the pressure readings from both pressure sensors (1, 1'), this embodiment allows computing the blood pressure difference, either instantaneous or averaged during the cardiac cycle, and, under the assumption of known blood flow, this allows computing the hemodynamic resistance, which enables quantifying and monitoring the evolution of restenosis and the impact of therapeutic measures taken to arrest or revert it.

The invention is defined in the appended claims.

**Claims**

1. A pressure sensor (1), adapted to be implanted inside a human or animal vessel (2) for measuring intravascular pressure, comprising:

   - a hermetic capsule (3) comprising at least a partially flexible portion (3');
   - an electronic circuit (4) housed in the capsule (3), said electronic circuit (4) being adapted to generate an electrical transduction pressure signal resulting from a deformation of the partially flexible portion (3') of the capsule (3), and dependent on the absolute pressure outside the capsule (3);
   - an electrode pair (5, 5') electrically connected to the electronic circuit (4), wherein each electrode of the electrode pair (5, 5') is, at least in part, arranged externally to the capsule (3);

   **wherein** the electronic circuit (4) comprises:

   - a digital control unit (29);
   - an interrogation stage (25) adapted to receive an interrogation signal transmitted by volume conduction to the electrode pair (5, 5'); to process said interrogation signal by means of the digital control unit (29); to generate, in response to the interrogation signal, the electrical transduction pressure signal; and to transmit, in response to the interrogation signal, said electrical transduction pressure signal to a medium (15) by volume conduction, through the electrode pair (5, 5');
   - a power transfer stage (23) adapted to receive at least part of the interrogation signal transmitted by volume conduction to the electrode pair (5, 5'), and to transfer the power associated to said at least part of the interrogation signal to the digital control unit (29) and to the interrogation stage (25).

2. The pressure sensor (1) according to the preceding claim, further comprising fixation means (12) adapted for attaching the sensor (1) to a vessel (2), wherein the fixation means (12) comprise at least one electrode of the electrode pair (5, 5').

3. The pressure sensor (1) according to claim 2, wherein the fixation means (12) comprise at least one of the following: a stent structure (16, 16', 16"), or a wire loop structure (11).

4. The pressure sensor (1) according to any of claims 2-3, wherein an electrode of the electrode pair (5,5') comprises a cable structure (17).

5. The pressure sensor (1) according to any of the preceding claims, wherein the capsule (3) body is made, at least in part, of metallic material.

6. The pressure sensor (1) according to any of the preceding claims, further comprising a pressure transmission fluid housed inside the capsule (3), wherein the fluid is arranged between the flexible portion (3') of the capsule (3) and the electronic circuit (4), and wherein the electrical circuit (4) further comprises a pressure transducer.

7. The pressure sensor (1) according to claims 1-5, wherein the partially flexible portion (3') of the capsule (3) is electrically connected to the electronic circuit (4) and capacitively coupled to said electronic circuit (4) through a conductor (19), forming a pressure transducer whose capacitance depends on the deformation of the partially flexible portion (3') of the capsule (3).

8. The pressure sensor (1) according to claims 1-5, wherein said sensor (1) comprises two conductors (19, 19'), and wherein the electronic circuit (4) is capacitively coupled to the capsule (3) through said conductors (19, 19'), forming two pressure transducers connected in series.

9. The pressure sensor (1) according to any of the preceding claims, wherein the capsule (3) and/or the electrode pair (5, 5') are, at least in part, covered with insulating material.

10. The pressure sensor (1) according to any of the preceding claims, wherein the power transferring stage (9) of the electronic circuit (4) comprises a blocking capacitor (24) connected in series with the electrode pair (5, 5'), adapted so as to prevent passage of dc current and to enable the flow of high frequency current to and from the electronic circuit (4); and

    wherein the interrogation stage (25) comprises a demodulator unit (26) adapted to compare low-pass filtered signals obtained with a rectifier (22) and a digital converter (27) connected to a sensing capacitor (28) or to a piezoresistive pressure sensor; and
    wherein the digital control unit (29) is adapted to generate an electrical transduction pressure signal by modulating the load of the pressure sensor (1) and reading it with the digital converter (27).

11. A system comprising one or more pressure sensors (1) according to any of the preceding claims and a reading unit (6), wherein said reading unit (6) comprises:

    - a signal generator (10) adapted to generate an interrogation signal; and to transmit said interrogation signal to the pressure sensors (1) through a medium (15) by volume conduction;
    - receiving means (20) adapted to receive an electrical transduction pressure signal from the pressure sensor (1) through the medium (15) by volume conduction; and
    - processing means (21) adapted to process information associated to the electrical transduction pressure signal.

12. The system according to the preceding claim, wherein the reading unit (6) is adapted to be implanted inside a human or animal body.

13. An interrogation and powering method for operating a system according to any of claims 11-12 to obtain pressure measurements,
**characterised in that** it comprises the following steps:

    - generating, with the reading unit (6), an interrogation signal and transmitting said interrogation signal to at least one pressure sensor (1) through a medium (15), by volume conduction;

- receiving the interrogation signal in the pressure sensor (1);
- transferring power associated to at least part of the interrogation signal to the digital control unit (29) and to the interrogation stage (25) of the electronic circuit (4) of the pressure sensor (1);
- processing said interrogation signal by means of the digital control unit (29) of the electronic circuit (4) of the pressure sensor (1);
- generating, in response to the interrogation signal, an electrical transduction pressure signal resulting from the deformation of the partially flexible portion (3') of the capsule (3), by means of the interrogation stage (25) of the electronic circuit (4) of the pressure sensor (1);
- transmitting the electrical transduction pressure signal to the reading unit (6) through the medium (15) by volume conduction, by means of the interrogation stage (25) of the electronic circuit (4) of the pressure sensor (1).

14. The method according to the preceding claim, wherein the interrogation signal generated by the reading unit (6) is a high-frequency alternating current comprising one or more bursts which encode the address of at least one pressure sensor (1) and wherein said current reaches the at least one pressure sensor (1) by volume conduction through the medium (15).

15. The method according to any of claims 13-14, wherein the step of transmitting the electrical transduction pressure signal comprises modulating the load that the at least one pressure sensor (1) exhibits to the passage of high-frequency current flowing through the medium (15) by volume conduction.

**Patentansprüche**

1. Drucksensor (1), welcher dazu angepasst ist, innerhalb eines menschlichen oder tierisches Gefäßes (2) zur Messung des intravaskulären Drucks implantiert zu werden, umfassend:

   - eine hermetische Kapsel (3), welche mindestens einen teilweise flexiblen Abschnitt (3') umfasst;
   - eine elektronische Schaltung (4), welche in der Kapsel (3) aufgenommen ist, wobei die besagte elektronische Schaltung (4) dazu angepasst ist, ein elektrisches Transduktionsdrucksignal zu erzeugen, welches sich aus einer Verformung des teilweise flexiblen Abschnitts (3') der Kapsel (3) ergibt und vom Absolutdruck außerhalb der Kapsel (3) abhängig ist;
   - ein Paar von Elektroden (5, 5'), welche mit der elektronischen Schaltung (4) elektrisch verbunden sind, wobei jede Elektrode des Paars von Elektroden (5, 5'), mindestens zum Teil, äußerlich zur Kapsel (3) angeordnet ist;

   **wobei** die elektronische Schaltung (4) Folgendes umfasst:

   - eine digitale Steuereinheit (29);
   - eine Abfragestufe (25), welche dazu angepasst ist, ein Abfragesignal zu empfangen, welches mittels Volumenleitung auf das Paar von Elektroden (5, 5') weitergeleitet wird; das besagte Abfragesignal mittels der digitalen Steuereinheit (29) zu verarbeiten; als Antwort auf das Abfragesignal, das elektrische Transduktionsdrucksignal zu erzeugen; und, als Antwort auf das Abfragesignal, das besagte elektrische Transduktionsdrucksignal auf ein Medium (15) mittels Volumenleitung, über das Paar von Elektroden (5, 5'), weiterzuleiten;
   - eine Leistungsübertragungsstufe (23), welche dazu angepasst ist, mindestens ein Teil des Abfragesignales, welches mittels Volumenleitung auf das Paar von Elektroden (5, 5') weitergeleitet wird, zu empfangen, und die Leistung, welche mit dem besagten mindestens einen Teil des Abfragesignals assoziiert ist, auf die digitale Steuereinheit (29) und auf die Abfragestufe (25) zu übertragen.

2. Drucksensor (1) nach dem vorhergehenden Anspruch, zusätzlich umfassend Fixierungsmittel (12), welche dazu angepasst sind, den Sensor (1) an einem Gefäß (2) zu befestigen, wobei die Fixierungsmittel (12) mindestens eine Elektrode des Paars von Elektroden (5, 5') umfassen.

3. Drucksensor (1) nach Anspruch 2, wobei die Fixierungsmittel (12) mindestens eine der folgenden umfassen: eine Stentstruktur (16, 16', 16"), oder eine Drahtschlaufenstruktur (11).

4. Drucksensor (1) nach einem der Ansprüche 2-3, wobei eine Elektrode des Paars von Elektroden (5, 5') eine Kabelstruktur (17) umfasst.

5. Drucksensor (1) nach einem der vorhergehenden Ansprüche, wobei der Körper der Kapsel (3), mindestens zum Teil, aus metallischem Material hergestellt ist.

6. Drucksensor (1) nach einem der vorhergehenden Ansprüche, zusätzlich umfassend ein Druckübertragungsfluid, welches innerhalb der Kapsel (3) aufgenommen ist, wobei das Fluid zwischen dem flexiblen Abschnitt (3') der Kapsel (3) und der elektronischen Schaltung (4) angeordnet ist, und wobei die elektrische Schaltung (4) zusätzlich einen Druckwandler umfasst.

7. Drucksensor (1) nach den Ansprüchen 1-5, wobei der teilweise flexible Abschnitt (3') der Kapsel (3) mit der elektronischen Schaltung (4) elektrisch verbunden ist und mit der besagten elektronischen Schaltung (4) über einen Leiter (19) kapazitiv gekoppelt ist, unter Bildung eines Druckwandlers, dessen Kapazität von der Verformung des teilweise flexiblen Abschnitts (3') der Kapsel (3) abhängig ist.

8. Drucksensor (1) nach den Ansprüchen 1-5, wobei der besagte Sensor (1) zwei Leiter (19, 19') umfasst, und wobei die elektronische Schaltung (4) mit der Kapsel (3) über die besagten Leiter (19, 19') kapazitiv gekoppelt ist, unter Bildung zweier Druckwandler, welche in Reihe geschaltet sind.

9. Drucksensor (1) nach einem der vorhergehenden Ansprüche, wobei die Kapsel (3) und/oder das Paar von Elektroden (5, 5'), mindestens zum Teil, mit Isolierungsmaterial gedeckt sind.

10. Drucksensor (1) nach einem der vorhergehenden Ansprüche, wobei die Leistungsübertragungsstufe (9) der elektronischen Schaltung (4) einen Blockkondensator (24) umfasst, welcher mit dem Paar von Elektroden (5, 5') in Reihe geschaltet ist und dazu angepasst ist, um den Durchgang von Gleichstrom zu verhindern und ein Fluss von Hochfrequenzstrom zu und von der elektronischen Schaltung (4) zu ermöglichen; und

wobei die Abfragestufe (25) eine Demodulatoreinheit (26) umfasst, welche dazu angepasst ist, Tiefpassfiltersignale zu vergleichen, welche mit einem Gleichrichter (22) und einem Digitalumsetzer (27), welche mit einem Messkondensator (28) oder mit einem piezoresistiven Drucksensor verbunden sind, erhalten werden; und wobei die digitale Steuereinheit (29) dazu angepasst ist, ein elektrisches Transduktionsdrucksignal mittels der Modulation der Last des Drucksensors (1) und des Ablesens desselben mit dem Digitalumsetzer (27) zu erzeugen.

11. System umfassend einen oder mehrere Drucksensoren (1) nach einem der vorhergehenden Ansprüche und eine Ableseeinheit (6), wobei die besagte Ableseeinheit (6) Folgendes umfasst:

- einen Signalgenerator (10), welcher dazu angepasst ist, ein Abfragesignal zu erzeugen; und das besagte Abfragesignal auf die Drucksensoren (1) über ein Medium (15) mittels Volumenleitung weiterzuleiten;
- Empfangsmittel (20), welche dazu angepasst sind, an elektrisches Transduktionsdrucksignal vom Drucksensor (1) über das Medium (15) mittels Volumenleitung zu empfangen; und
- Verarbeitungsmittel (21), welche dazu angepasst sind, Information, welche mit dem elektrischen Transduktionsdrucksignal assoziiert ist, zu verarbeiten.

12. System nach dem vorhergehenden Anspruch, wobei die Ableseeinheit (6) dazu angepasst ist, innerhalb eines menschlichen oder tierischen Körpers implantiert zu werden.

13. Abfrage- und Antriebverfahren zum Betrieb eines Systems nach einem der Ansprüche 11-12, um Druckmessungen zu erhalten,
**dadurch gekennzeichnet, dass** es die folgenden Schritte umfasst:

- das Erzeugen, mit der Ableseeinheit (6), eines Abfragesignals und das Weiterleiten des besagten Abfragesignals auf mindestens einen Drucksensor (1) über ein Medium (15), mittels Volumenleitung;
- das Empfangen des Abfragesignals im Drucksensor (1);
- das Übertragen der Leistung, welche mit mindestens einem Teil des Abfragesignals assoziiert ist, auf die digitale Steuereinheit (29) und auf die Abfragestufe (25) der elektronischen Schaltung (4) des Drucksensors (1);
- das Verarbeiten des besagten Abfragesignals mittels der digitalen Steuereinheit (29) der elektronischen Schaltung (4) des Drucksensors (1);
- das Erzeugen, als Antwort auf das Abfragesignal, eines elektrischen Transduktionsdrucksignals, welches sich aus der Verformung des teilweise flexiblen Abschnitts (3') der Kapsel (3) ergibt, mittels der Abfragestufe (25) der elektronischen Schaltung (4) des Drucksensors (1);

- das Weiterleiten des elektrischen Transduktionsdrucksignals auf die Ableseeinheit (6) über das Medium (15) mittels Volumenleitung, mittels der Abfragestufe (25) der elektronischen Schaltung (4) des Drucksensors (1).

14. Verfahren nach dem vorhergehenden Anspruch, wobei das von der Ableseeinheit (6) erzeugte Abfragesignal ein Hochfrequenz-Wechselstrom ist, welcher einen oder mehrere Stöße umfasst, welche die Adresse mindestens eines Drucksensors (1) kodieren, und wobei der besagte Strom den mindestens einen Drucksensor (1) mittels Volumenleitung über das Medium (15) erreicht.

15. Verfahren nach einem der Ansprüche 13-14, wobei der Schritt des Weiterleitens des elektrischen Transduktionsdrucksignals das Modulieren der Last, welche der mindestens eine Drucksensor (1) aufweist, zum Durchgang von Hochfrequenzstrom, welcher über das Medium (15) mittels Volumenleitung fließt, umfasst.

**Revendications**

1. Capteur de pression (1), adapté pour être implanté à l'intérieur d'un vaisseau humain ou animal (2) pour mesurer la pression intravasculaire, comprenant :

   - une capsule hermétique (3) comprenant au moins une partie partiellement flexible (3') ;
   - un circuit électronique (4) logé dans la capsule (3), ledit circuit électronique (4) étant adapté pour générer un signal de pression de transduction électrique résultant d'une déformation de la partie partiellement flexible (3') de la capsule (3), et dépendant de la pression absolue à l'extérieur de la capsule (3) ;
   - une paire d'électrodes (5, 5') connectées électriquement au circuit électronique (4), dans lequel chaque électrode de la paire d'électrodes (5, 5') est, au moins en partie, disposée à l'extérieur de la capsule (3) ;

   **dans lequel** le circuit électronique (4) comprend

   - une unité de commande numérique (29) ;
   - un étage d'interrogation (25) adapté pour recevoir un signal d'interrogation transmis par conduction de volume à la paire d'électrodes (5, 5') ; pour traiter ledit signal d'interrogation au moyen de l'unité de commande numérique (29) ; pour générer, en réponse au signal d'interrogation, le signal de pression de transduction électrique ; et pour transmettre, en réponse au signal d'interrogation, ledit signal de pression de transduction électrique à un milieu (15) par conduction de volume, à travers la paire d'électrodes (5, 5') ;
   - un étage de transfert de puissance (23) adapté pour recevoir au moins une partie du signal d'interrogation transmis par conduction de volume à la paire d'électrodes (5, 5'), et pour transférer la puissance associée à ladite au moins une partie du signal d'interrogation à l'unité de commande numérique (29) et à l'étage d'interrogation (25).

2. Capteur de pression (1) selon la revendication précédente, comprenant en outre des moyens de fixation (12) adaptés pour attacher le capteur (1) à un vaisseau (2), dans lequel les moyens de fixation (12) comprennent au moins une électrode de la paire d'électrodes (5, 5').

3. Capteur de pression (1) selon la revendication 2, dans lequel les moyens de fixation (12) comprennent au moins l'un des éléments suivants : une structure de stent (16, 16', 16") ou une structure de boucle de fil (11).

4. Capteur de pression (1) selon l'une quelconque des revendications 2-3, dans lequel une électrode de la paire d'électrodes (5, 5') comprend une structure de câble (17).

5. Capteur de pression (1) selon l'une quelconque des revendications précédentes, dans lequel le corps de la capsule (3) est constitué, au moins en partie, d'un matériau métallique.

6. Capteur de pression (1) selon l'une quelconque des revendications précédentes, comprenant en outre un fluide de transmission de pression logé à l'intérieur de la capsule (3), dans lequel le fluide est disposé entre la partie flexible (3') de la capsule (3) et le circuit électronique (4), et dans lequel le circuit électrique (4) comprend en outre un transducteur de pression.

7. Capteur de pression (1) selon les revendications 1-5, dans lequel la partie partiellement flexible (3') de la capsule (3) est connectée électriquement au circuit électronique (4) et couplée capacitivement audit circuit électronique (4) à travers un conducteur (19), formant un transducteur de pression dont la capacité dépend de la déformation de la partie partiellement flexible (3') de la capsule (3).

8. Capteur de pression (1) selon les revendications 1-5, dans lequel ledit capteur (1) comprend deux conducteurs (19, 19'), et dans lequel le circuit électronique (4) est couplé capacitivement à la capsule (3) à travers lesdits conducteurs (19, 19'), formant ainsi deux transducteurs de pression connectés en série.

9. Capteur de pression (1) selon l'une quelconque des revendications précédentes, dans lequel la capsule (3) et/ou la paire d'électrodes (5, 5') sont, au moins en partie, recouvertes d'un matériau isolant.

10. Capteur de pression (1) selon l'une quelconque des revendications précédentes, dans lequel l'étage de transfert de puissance (9) du circuit électronique (4) comprend un condensateur de blocage (24) connecté en série avec la paire d'électrodes (5, 5'), adapté de manière à empêcher le passage du courant cc et à permettre la circulation du courant à haute fréquence vers et depuis le circuit électronique (4) ; et

    dans lequel l'étage d'interrogation (25) comprend une unité de démodulation (26) adaptée pour comparer des signaux filtrés passe-bas obtenus avec un redresseur (22) et un convertisseur numérique (27) connecté à un condensateur de détection (28) ou à un capteur de pression piézorésistif ; et
    dans lequel l'unité de commande numérique (29) est adaptée pour générer un signal de pression de transduction électrique en modulant la charge du capteur de pression (1) et en le lisant à l'aide du convertisseur numérique (27).

11. Système comprenant un ou plusieurs capteurs de pression (1) selon l'une quelconque des revendications précédentes et une unité de lecture (6), dans lequel ladite unité de lecture (6) comprend :

    - un générateur de signaux (10) adapté pour générer un signal d'interrogation ; et pour transmettre ledit signal d'interrogation aux capteurs de pression (1) à travers un milieu (15) par conduction de volume ;
    - des moyens de réception (20) adaptés pour recevoir un signal de pression de transduction électrique provenant du capteur de pression (1) à travers le milieu (15) par conduction de volume ; et
    - des moyens de traitement (21) adaptés pour traiter les informations associées au signal de pression de transduction électrique.

12. Système selon la revendication précédente, dans lequel l'unité de lecture (6) est adaptée pour être implantée à l'intérieur d'un corps humain ou animal.

13. Procédé d'interrogation et d'alimentation pour faire fonctionner un système selon l'une quelconque des revendications 11-12 afin d'obtenir des mesures de pression,
    **caractérisé en ce qu'**il comprend les étapes suivantes :

    - générer, avec l'unité de lecture (6), un signal d'interrogation et transmettre ledit signal d'interrogation à au moins un capteur de pression (1) à travers un milieu (15), par conduction de volume ;
    - recevoir le signal d'interrogation dans le capteur de pression (1) ;
    - transférer la puissance associée à au moins une partie du signal d'interrogation à l'unité de commande numérique (29) et à l'étage d'interrogation (25) du circuit électronique (4) du capteur de pression (1) ;
    - traiter ledit signal d'interrogation au moyen de l'unité de commande numérique (29) du circuit électronique (4) du capteur de pression (1) ;
    - générer, en réponse au signal d'interrogation, un signal de pression de transduction électrique résultant de la déformation de la partie partiellement flexible (3') de la capsule (3), au moyen de l'étage d'interrogation (25) du circuit électronique (4) du capteur de pression (1) ;
    - transmettre le signal de pression de transduction électrique à l'unité de lecture (6) à travers le milieu (15) par conduction de volume, au moyen de l'étage d'interrogation (25) du circuit électronique (4) du capteur de pression (1).

14. Procédé selon la revendication précédente, dans lequel le signal d'interrogation généré par l'unité de lecture (6) est un courant alternatif à haute fréquence comprenant une ou plusieurs salves qui codent l'adresse d'au moins un capteur de pression (1) et dans lequel ledit courant atteint l'au moins un capteur de pression (1) par conduction de volume à travers le milieu (15).

15. Procédé selon l'une quelconque des revendications 13 à 14, dans lequel l'étape de transmission du signal de pression de transduction électrique comprend la modulation de la charge que l'au moins un capteur de pression (1) présente au passage du courant à haute fréquence circulant dans le milieu (15) par conduction de volume.

**FIG. 1**

**FIG. 2**

**FIG. 3**

**FIG. 4**

**FIG. 5**

FIG. 6

FIG. 7

FIG. 8

FIG. 9

FIG. 10

**blood flow**

2    16'    1    16    1'    16"

## FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017200769 A2
- US 7353711 B1
- US 20160029956 A1
- WO 2006105245 A2
- US 8515559 B2

**Non-patent literature cited in the description**

- **LIU et al.** A Single-Connector Stent Antenna for Intravascular Monitoring Applications. *Sensors,* vol. 19 (21), 4616
- **L. BECERRA FAJARDO et al.** *Microcontrolled injectable stimulators based on electronic rectification of high frequency current bursts,* 2016